(19) Europäisches Patentamt
European Patent Office
Office européen des brevets

(11) EP 0 332 379 B1

(12) EUROPEAN PATENT SPECIFICATION

(45) Date of publication and mention of the grant of the patent:
**14.08.1996 Bulletin 1996/33**

(51) Int Cl.[6]: **C12P 13/04**, C12P 17/00, C12P 41/00

(21) Application number: **89302231.9**

(22) Date of filing: **06.03.1989**

(54) **Production process of L-alpha-amino acids**

Verfahren zur Herstellung von L-alpha-Aminosäuren

Procédé de préparation de L-alpha-acides aminés

(84) Designated Contracting States:
**BE CH DE FR GB IT LI NL**

(30) Priority: **08.03.1988 JP 52694/88**
**08.03.1988 JP 52695/88**
**09.03.1988 JP 55781/88**

(43) Date of publication of application:
**13.09.1989 Bulletin 1989/37**

(73) Proprietor: **JAPAN ENERGY CORPORATION**
**Minato-ku, Tokyo (JP)**

(72) Inventors:
- **Wakamoto, Akiko c/o Nippon Mining Toda-shi Saitama-ken (JP)**
- **Takahashi, Osamu c/o Nippon Mining Toda-shi Saitama-ken (JP)**
- **Furuhashi, Keizo c/o Nippon Mining Toda-shi Saitama-ken (JP)**

(74) Representative: **O'Brien, Caroline Jane et al**
**MEWBURN ELLIS**
**York House**
**23 Kingsway**
**London WC2B 6HP (GB)**

(56) References cited:
**WO-A-86/07386** **FR-A- 2 245 585**

- **CHEMICAL ABSTRACTS, vol. 105, no. 7, August 1986, Columbus, OH (US); S.Y. CHOI et al., p. 513, no. 59419h**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 332 379 B1

## Description

This invention relates to a process for the production of L-α-amino acids from nitrile compounds, particularly from racemic α-aminonitrile compounds or derivatives thereof by making use of microorganisms.

L-α-amino acids are important chemical substances useful in a variety of fields such as foods, feeds, pharmaceuticals and cosmetics.

Conventionally, the fermentation, synthetic, enxymatic and extraction processes have been known to produce L-α-amino acids. Among these processes, the synthetic process comprises synthesizing first DL-α-amino acids in accordance with the Strecker process or the modification process thereof and then subjecting the amino acids to optical resolution to produce L-α-amino acids. In the optical resolution, the enzymatic process using aminoacylase or similar processes have been employed (Kagaku Zokan 97, Progress of Asymmetric Synthesis and Optical Resolution, Kagakudojin, 1982, p. 175).

However, production of L-α-amino acids according to the above-described synthetic process requires increased costs in the stage of optical resolution. For such economical reasons, the process has recently suffered gradual share reduction in the production of amino acids.

An attempt has also been made reportedly to produce α-amino acids from DL-α-aminonitriles, which are intermediates in the synthesis of α-amino acids according to the aforementioned Strecker process and represented by the following general formula:

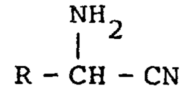

wherein R is an alkyl group, phenyl group or the like, by making use of microorganisms [Y. Fukuda et al., J. Ferment. Technol. 49, 1101 (1971)].

According to this report, however, DL-α-aminopropionitrile and DL-α-aminoisovaleronitrile are hydrolyzed in the presence of microorganisms belonging to Corynebacterium sp. to produce respectively DL-alanine and DL-valine, and therefore L-α-amino acids can not be obtained directly.

It has also been reported that amino acids are produced by the hydrolysis of DL-α-aminonitriles in the presence of Brevibacterium sp. R312 [J. C. Jallageas et al., Adv. Biochem. Engineer, 14, 1 (1980)]. According to the report, only DL-α-amino acids are obtainable from DL-α-aminonitriles. In this connection, although the above report discloses simultaneous production of L-α-amino-acids and D-α-amino acid amides from DL-α-aminonitriles by the use of Brevibacterium sp. A4, a mutant obtained from Brevibacterium sp. B312, no report has been found with regard to the direct and exclusive production of L-α-amino acids from DL-α-aminonitriles. Further, this process necessarily involves such complex operation of separating an L-α-amino acid from a D-α-amino acid amide and hence is unfavoured.

It has also been disclosed to produce amino acids by irradiating light to the reaction system before completion of the microbiological hydration of DL-α-aminonitrile compounds (Japanese Patent Laid-Open No. 162191/1986). However, only DL-α-amino acids are obtainable by this process.

The use of enantio-preferential enzymes for the conversion of amino nitriles into the corresponding amino acid amides or amino acid, has been disclosed in International Patent Application WO86/07386. The process described in this application involves the conversion of amino nitriles into the corresponding amino acid amides in an enzyme catalysed reaction using a nitrilase which preferentially converts one of the two enantiomeric forms of the amino nitriles into the corresponding amino acid amides. The amino acid amides of said enantiomeric form thus generated is subsequently hydrolysed by chemical means to provide the amino acid of the corresponding enantiomeric form. In the process disclosed in this application, the enzyme-catalyzed reaction is carried out at a pH value of about 7.

Thus, no report has been made to produce L-α-amino acids directly from racemic α-aminonitrile compounds.

In view of the aforementioned existing state of art, the present inventors have made intensive investigations and found that L-α-amino acids are selectively produced when microorganisms having the ability of nitrile hydrolysis are caused to act on an α-aminonitrile compound at a pH in the range of 8-12 or on an α-(N-alkylideneamino) nitrile comopound. The present invention has been completed on the basis of this finding and makes it the object to provide a process for the direct production of L-α-amino acids from α-aminonitrile compounds or derivatives thereof by making use of microorganisms selected from specific genera and having nitrile-hydrolyzing ability.

The process of the present invention comprises causing a microorganism having nitrile-hydrolyzing activity to act on one or more α-aminonitrile compounds represented by the following general formula (I):

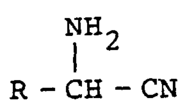

(I)

and/or the following general formula (II):

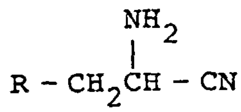

(II)

at a pH in the range of 8-12 and/or in the presence of an aldehyde, or on one or more α-(N-alkylideneamino)nitrile compounds represented by the following general formula (III):

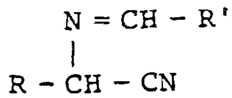

(III)

and/or the following general formula (IV):

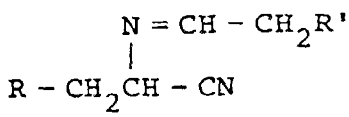

(IV)

to convert the compound or compounds to L-α-amino acids, R and R' in the above formulae being individually an alkyl group, substituted alkyl group, phenyl group, substituted phenyl group, imidazolyl group, substituted imidazolyl group, indolyl group, substituted indolyl group, furyl group, substituted furyl group, pyridyl group, substituted pyridyl group, thiazolyl group or substituted thiazolyl group, and R and R' may be the same or different groups.

DETAILED DESCRIPTION OF THE PREFERRED EMBODIMENTS OF THE INVENTION:

The microorganisms useful in the practice of the present invention are, as described above, those selected from the group having nitrile-hydrolyzing ability and may be illustrated as in the following Table 1.

Table 1

| Strain | FERM-BP No. |
|---|---|
| Nocardia sp. PC-29 | 1561 |
| Nocardia sp. PA-34 | 1559 |
| Nocardia sp. AB-16 | 1555 |
| Nocardia sp. BA-1 | 1557 |
| Mycobacterium sp. AB-43 | 1556 |
| Corynebacterium sp. PA-15 | 1558 |
| Corynebacterium sp. PC-3 | 1560 |

These microorganisms have been deposited to Fermentation Research Institute of the Agency of Industrial Science and Technology under the Budapest Treaty on Nov. 5, 1987 with Accession Numbers given in Table 1.

The properties of the above-described microorganisms are illustrated in Table 2.

Table 2

| Strain / Properties | Nocardia sp. | | | |
|---|---|---|---|---|
| | PC-29 | PA-34 | AB-16 | BA-1 |
| Shape | sphere | rod | sphere | sphere |
| Motility | no | no | no | no |
| Spore | no | no | no | no |
| Gram stain | + | + | + | + |
| Oxidase | - | - | - | - |
| Catalase | + | + | + | + |
| O-F test | F | F | N | N |
| Gas generation from glucose | - | - | - | - |
| Acid formation from carbohydrate | | | | |
| arabinose | - | + | - | - |
| glucose | - | - | - | - |
| lactose | - | + | - | - |
| maltose | - | + | - | - |
| mannit | - | + | - | - |
| raffinose | - | + | - | - |
| saccharose | - | + | - | - |
| xylose | - | + | - | - |
| glycerol | - | + | - | - |
| VP reaction | + | + | + | + |
| MR reaction | - | - | - | - |
| Growth in 6.5% NaCl | - | + | + | - |
| Reduction of nitrate | + | + | + | ± |
| Utilization of citric acid | + | - | + | - |
| Hydrolysis of aesculin | + | - | - | - |
| Liquefaction of gelatin | - | - | - | - |
| Color of colony | yellow | cinnabar | pink | cinnabar |

Table 2 (Cont'd.)

| *Mycobacterium* sp. | *Corynebacterium* sp. | |
|---|---|---|
| AB-43 | PA-15 | PC-3 |
| rod | rod | rod |
| no | no | no |
| no | no | no |
| + | + | + |
| - | - | - |
| + | + | + |
| N | O | O |
| - | - | - |
| +<br>-<br>-<br>-<br>-<br>-<br>-<br>±<br>- | -<br>±<br>-<br>-<br>-<br>-<br>-<br>-<br>- | -<br>-<br>-<br>-<br>-<br>-<br>-<br>-<br>- |
| + | + | + |
| - | - | - |
| - | + | + |
| + | + | + |
| - | - | - |
| - | + | + |
| - | + | + |
| Yellow | Yellow | Yellow |

F, Fermentation;
O, Oxidation;
N, not determined

As seen in Table 2, the microorganisms used in the present invention are all Gram-positive aerobic bacteria and

hence do not grow at all in anaerobic conditions. They do not exhibit motility, catalases being positive, and they do not form heat-resistant spores. They show mycelial growth in the early stage of culture. Branching cells and cocci are also observed in older cultures. As to the utilization of sugar, they do not generate gas from sugar, and their acid-forming ability is weak so that when they are incubated in litmus milk, the milk will turn blue indicating alkaline.

Among the above-described microorganisms, Nocardia sp. PC-29 forms somewhat dry and wrinkled colonies on nutrient agar and shows markedly branched mycelial growth in the early stage of culture. It grows at 5 - 32°C but does not grow at 37°C.

On the other hand, Nocardia sps. PA-34, AB-16 and BA-1 do not grow at 5 - 10°C, but grow at 37 - 42°C. They show mycelial growth in the early stage of culture and in older cultures shorter rods or cocci are formed.

Mycobacterium sp. AB-43 is positive in acid-fast stain and does not grow at 45°C, forming viscous yellow-orange colonies. Corynebacterium sps. PA-15 and PC-3 are Gram-positive rods and their shapes are rather 5 irregular. In older cultures, they form granules in the cells and exhibit a negative Gram stain, and shorter rods or cocci are formed. The strains grow at 5 - 10°C, but do not grow at 37°C or higher. The colonies on nutrient agar are yellow-green and creamy, and have strong abilities of hydrolyzing both gelatin and starch but no ability of decomposing cellulose.

The above-described microorganisms were identified according to Bergey's Manual of Systematic Bacteriology, 1986, in view of the properties shown in Table 2 and as described above.

In the present invention, the α-aminonitrile compounds represented by the foregoing general formulae (I) and (II), the substrates for producing L-α-amino acids by the aforesaid microorganisms, may be synthesized with ease according to the synthetic process of α-aminonitriles, i.e., the first stage reaction in the Strecker process [for example, J. Ferment. Technol., 49 1011 (1971) or Chem. Lett., 687 (1987)], or to the process disclosed in Org. Syn. Col. Vols. I, p. 21 and III, p. 84.

On the other hand, the α-(N-alkylideneamino)nitrile compounds represented by the general formulae (III) and (IV), which are another substrate, may be obtained in such a way that as soon as the aldehyde used as a raw material is confirmed to disappear by the analysis using gas chromatography in the synthesis of α-aminonitrile compounds or in the first stage reaction in the Strecker process, inorganic salts are separated out by such a procedure as filtration or extraction and the resulting solution is concentrated. The crude products may in some cases contain, as impurities, α-aminonitriles, which are the partial hydrolysis products of α-(N-alkylideneamino)nitrile compounds represented by the foregoing general formulae (III) and (IV), and aldehydes used as a raw material for the synthesis. After these impurifies have been removed by such a procedure as fractional distillation, the resulting products are used as the substrate. However, the crude products containing the impurities may also be used as the substrate.

R and R' in the general formulae (I) (II) (III) and (IV) representing the α-aminonitrile compounds and derivatives thereof, i.e., α-(N-alkylideneamino)nitrile compounds, are individually an alkyl group, substituted alkyl group, phenyl group, substituted phenyl group, imidazolyl group, substituted imidazolyl group, indolyl group, substituted indolyl group, furyl group, substituted furyl group, pyridyl group, substituted pyridyl group, thiazolyl group or substituted thiazolyl group. No particular limitations are imposed on the substituents in the substituted alkyl group, substituted phenyl group, substituted imidazolyl group, substituted indolyl group, substituted furyl group, substituted pyridyl group and substituted thiazolyl group, among these Rs. However, as preferred substituents may be mentioned hydroxyl, methoxyl, mercapto, methylthio, amino, halogen, carboxyl, carboxamide, phenyl, hydroxyphenyl and guanyl by way of example.

Further, where the compounds of the general formulae (III) and (IV) are used as a substrate, use of the compound having the same groups for both R and R' as a raw material may lead to the formation of an L-α-amino acid, while use of the compound having different groups for R and R' as a raw material can result in the formation of a mixture of two L-α-amino acids.

It goes without saying that the aforesaid nitrile compounds and derivatives thereof used as the raw material may be used as a mixture of two or more of them without any inconvenience.

Illustrative examples of the above-described α-aminonitrile compound may include 2-aminopropanenitrile, 2-aminobutanenitrile, 2-amino-3-methylbutanenitrile, 2-amino-4-methylpentanenitrile, 2-amino-3-methylpentanenitrile, 2-amino-3-hydroxypropanenitrile, 2-amino-3-hydroxybutanenitrile, 2-amino-5-guanidinopentanenitrile, 2-amino-3-mercaptopropanenitrile, 2,7-diamino-4,5-dithiaoctanenitrile, 2-amino-4-methylthiobutanenitrile, 2-amino-4-methylthiopropanenitrile, 2-amino-3-phenylpropanenitrile, 3-(4-hydroxyphenyl)propanenitrile, 3-amino-3-cyanopropanoic acid, 4-amino-4-cyanobutanoic acid, 3-amino-3-cyanopropanamide, 4-amino-4-cyanobutanamide, 2,6-diaminohexanenitrile, 2,6-diamino-5-hydroxyhexanenitrile, 2-amino-3-(3-indolyl)propanenitrile, 2-amino-3-(4-imidazolyl)propanenitrile, 2-cyanopyrrolidine, 2-cyano-4-hydroxypyrrolidine and 2-amino-2-phenylethanenitrile.

On the other hand, as the α-(N-alkylideneamino)nitrile compounds may be mentioned the Schiff base compounds of the α-aminonitrile compounds as illustrated above with the aldehydes which are the raw material for the synthesis of the α-aminonitriles.

In the present invention, where the α-aminonitrile compound is used as a substrate, the substrate is brought into contact with a microorganism at a pH in the range of 8 - 12 or else it is brought into contact with a microorganism in the presence of an aldehyde. As the aldehyde used for this purpose, it is possible to use an aldehyde, which is the

raw material in the synthesis of the aminonitrile compounds represented by the general formula (I) or (II) illustrated above. In this case, solely an L-α-amino acid is obtained, whereas use of a different aldehyde may result in the formation of a mixture of different L-α-amino acids.

In the present invention, in order to produce an L-α-amino acid by causing each of the above-described microorganisms, which have nitrile-hydrolyzing activities, to act on the aforementioned nitrile compound or derivative thereof used as a raw material (hereinafter abbreviated as "the raw nitrile"), it is advisable to use any of the following processes (a) to (c).

Specifically, it is possible to employ (a) a process comprising incubating a microorganism in a medium containing a nitrile compound such as propionitrile to obtain a cell mass and bringing the thus-proliferated cell mass into contact with the raw nitrile or the raw nitrile plus an aldehyde to react them with each other, (b) a process comprising cultivating microorganism in advance, bringing the thus-proliferated cell mass into contact with a nitrile compound such as propionitrile, and adding to the resulting cell mass the raw nitrile or the raw nitrile plus an aldehyde to react them with each other, or (c) a process comprising cultivating a microorganism in advance, and bringing the thus-proliferated cell mass into direct contact with the raw nitrile or the raw nitrile plus an aldehyde to react them with each other.

In these reaction processes, it is also feasible to use destructed products of proliferated cells, dried cells, enzyme preparations such as cell free extract and purified nitrile-hydrolyzing enzymes, or cells and enzyme preparations immobilized according to conventional procedure.

The nitrile compounds useful in the practice of the processes (a) and (b) may embrace acetonitrile, n-butyronitrile, n-capronitrile, methacrylonitrile, isobutyronitrile, glutaronitrile, triacrylonitrile, crotononitrile, lactonitrile, succinonitrile, acrylonitrile, benzonitrile, phenylacetonitrile, etc. as well as propionitrile.

In the foregoing process (a), the preculture of each of the above-described microorganisms is inoculated to a medium which has been added with carbon sources such as glucose, sucrose, molasses and starchhydrolyzate, acetic acid and other utilizable carbon sources in addition to the nitrile compound, and further with a nitrogen source such as ammonium chloride, ammonium sulfate, ammonium phosphate, ammonium nitrate, urea, aqueous ammonia, sodium nitrate, and amino acids and other utilizable organic nitrogen compounds, inorganic salts such as calcium phosphate, sodium phosphate, magnesium sulfate, manganese sulfate, iron (II) sulfate, iron (III) chloride, calcium chloride and manganese chloride, salts of boric acid, salts of copper, zinc, etc., what is called micronutrients, and optionally growth-promoting materials such as vitamins, yeast extracts and corn steep liquor. The preculture is incubated therein under aerobic conditions to cause the cells to proliferate. The raw nitrile or the raw nitrile plus an aldehyde is added to the thus-obtained cultured broth, a suspension of cells separated from the cell-culture or enzyme preparations to react them with each other.

When an α-aminonitrile compound is reacted in the absence of an aldehyde, the reaction is carried out at a pH in the range of 8 - 12, whereas otherwise it is effected at a pH in the range of 4 - 13, preferably 8 - 12, for 1 - 6 days in both cases. If an α-aminonitrile compound is reacted at a pH outside the range of 8 - 12 in the absence of an aldehyde, an α-amino acid rich in L-configuration is formed, which is however low in optical purity and hence has no practical use. In this reaction, a variety of buffer solutions may be used. Of these, ammoniacal buffer solutions are preferred, including, for example, a mixture of aqueous ammonia and aqueous ammonium chloride solution, a mixture of aqueous ammonia and aqueous ammonium sulfate solution, a mixture of aqueous ammonia and aqueous ammonium phosphate solution, and a mixture of aqueous ammonia and aqueous ammonium acetate solution. Further, as the alkali for use to control pH, aqueous ammonia is preferred.

The reaction temperature should preferably fall within the range of 20 - 70°C. Further, the above-described carbon source, nitrogen source and other components used to proliferate the cells may be added properly to the reaction system to maintain and enhance the cell concentration or the cell's ability of nitrile hydrolysis. In the feeding of the raw nitrile, any one of the following methods may be employed: a method of adding at the time of reaction initiation, a method of intermittent addition or a method of continuous addition.

In the foregoing process (b), the nitrile compound is not added during incubation and proliferation of the cell mass as in the foregoing process (a), but is added after the cell mass has been proliferated so that the ability of nitrile hydrolysis of the mass of the microorganism is activated. Then, the cell mass is reacted with the raw nitrile or the raw nitrile plus an aldehyde to produce an L-α-amino acid.

In the foregoing process (c), as soon as the cell mass has been proliferated as in the process (b), the raw nitrile or the raw nitrile plus an aldehyde is added thereto for effecting reaction to produce an L-α-amino acid.

In either of the processes (b) and (c), it is possible to employ the same incubation conditions, reaction conditions and separation and collection procedures of the produced L-α-amino acid as those used in the process (a).

As regards the amount of an aldehyde to be used where the reaction is conducted in the presence of the aldehyde, it is preferable to use 0.1 - 10 mols, particularly 0.5 - 3 mols of the aldehyde per mol of an α-aminonitrile compound.

The L-α-amino acids formed by the above-described reactions are isolated by the use of well-known procedures such as phase separation, filtration, extraction and column chromatography.

The L-α-amino acids obtained according to the present invention as described above can find their applications

in a variety of fields including those of foods, feeds, pharmaceuticals and cosmetics.

As has been illustrated above, the present invention permits the direct and selective production of L-α-amino acids from racemic α-aminonitrile compounds or derivatives thereof by making use of microorganisms. Thus, the present invention is useful in the production of L-α-amino acids which are utilized in a variety of fields as described above.

The present invention will be illustrated more specifically by reference to the following examples. However, it should not be construed that the present invention is limited to or by the examples.

EXAMPLES:

Example 1:

(1) Preparation of medium:

A medium (100 ml) of the following composition was placed in a 500 ml Sakaguchi flask, which was then sterilized at 120°C for 20 minutes in an autoclave. To the medium was added 1 ml of propionitrile having been sterilized by means of a 0.2-μ Millipore Filter. The medium for the preparation of cell mass was thus obtained.

Medium composition:

| | |
|---|---|
| glucose | 10 g/l |
| yeast extract | 0.1 g/l |
| $Na_2HPO_4 \cdot 12H_2O$ | 2.5 g/l |
| $KH_2PO_4$ | 2.0 g/l |
| $MgSO_4 \cdot 7H_2O$ | 0.5 g/l |
| $FeSO_4 \cdot 7H_2O$ | 0.03 g/l |
| $CaCl_2 \cdot 2H_2O$ | 0.06 g/l |
| pH | 7.2 |

Strains for use:

| Name of strain | FERM-BP No. |
|---|---|
| Nocardia sp. PC-29 | 1561 |
| Nocardia sp. PA-34 | 1559 |
| Nocardia sp. AB-16 | 1555 |
| Nocardia sp. BA-1 | 1557 |
| Mycobacterium sp. AB-43 | 1556 |
| Corynbacterim sp. PA-15 | 1558 |
| Corynbacterium SP- PC-3 | 1560 |

(2) Incubation and proliferation of cell mass:

To the medium prepared as described above were inoculated 3 loopfuls of each of the five strains given in Table 3. The resultant medium was incubated under oscillation at 140 rpm at a temperature of 30°C for 48 hours.

The cell mass obtained by the incubation was isolated by centrifugation, washed once with a 0.1M $NH_4Cl$-$NH_3$ buffer solution (pH 10), and thereafter resuspended in a 0.1M $NH_4Cl$-$NH_3$ buffer solution so as to adjust the optical density (OD) at 40.

(3) Reaction:

Into a test tube with an inner diameter of 24 mm was admitted 5 ml of each of the cell suspensions obtained as described above. Then, 50 mg of DL-2-amino-2-phenylethanenitrile were added thereto and the test tube was stopcocked. The test tube was shaked at 300 rpm at a temperature of 30°C for 48 hours.

After completion of the reaction, the reaction mixture was extracted twice with 5 ml of hexane, and the water phase

thus-obtained was analyzed by thigh performance liquid chromatography (HPLC).

The quantity of L-α-phenylglycine thus-formed was determined by means of an amino acid analyzer (manufactured by Beckman Co.: Type 7300). Its absolute configuration and optical purity were determined by HPCL using CHIRALPAK WH (product of Daicel Chemical Industries, Ltd.) as a column packing. The results are as shown in Table 3.

Table 3

| Strain | L-α-phenylglycine | |
|---|---|---|
| | Amount (mg/ml) | Optical purity (% e.e.) |
| Nocardia sp. PC-29 | 0.4 | Not less than 99 |
| Nocardia sp. PA-34 | 0.2 | ditto |
| Nocardia sp. AB-16 | 0.1 | ditto |
| Nocardia sp. BA-1 | 0.1 | ditto |
| Mycobacterium sp. AB-43 | 0.5 | ditto |

Example 2:

In the same manner as in Example 1, the cell mass of Nocardia sp. PA-34 was prepared and collected, and then resuspended in a 0.1M $NH_4Cl$-$NH_3$ buffer solution so as to adjust the optical density (OD) at 40. Thereafter, 500 ml of the cell suspension thus-obtained was admitted in a 1.2-1 fermenter, to which 4.9 g (50 mmol) of DL-2-amino-3-methylbutanenitrile were added. While maintaining the pH at 10.0 ± 0.1 with 4N aqueous ammonia and 1N HCl, the resulting suspension was submitted to reaction at a temperature of 30°C and at 700 rpm agitation for 48 hours.

After completion of the reaction, the reaction mixture was centrifuged to obtain a supernatant, which was then filtered by means of a 0.45-μ Millipore Filter. The resulting water phase was analyzed by HPLC.

The accumulated concentration of L-valine was 0.5 mg/ml, while the optical purity thereof was 67% e.e. The quantity of L-valine was determined by HPLC using Asahipak Inertsil ODS (product of Asahi Chemical Industry Company, Ltd.) as a column packing. Its absolute configuration and optical purity were determined similarly by HPLC using CHIRALPAK WH (product of Daicel Chemical Industries, Ltd.)

Comparative Example 1:

Reaction and analysis were conducted by using the same cell mass and in the same manner as described in Example 2 except that the pH was adjusted at 7.0 ± 0.1 with 4N aqueous ammonia and 1N HCl in the procedure described in Example 2. As a consequence, the accumulated concentration of L-valine was found to be 3.5 mg/ml, while its optical purity was 32% e.e.

Comparative Example 2:

The cell mass of Nocardia sp. PA-34 was prepared in the same manner as described in Example 1. After the mass had been collected by centrifugation, it was washed twice with a 0.1M $Na_2HPO_4$-$KH_2PO_4$ buffer solution (pH 7.0) and then resuspended in a 0.1M $Na_2HPO_4$-$KH_2PO_4$ buffer solution so as to adjust the optical density (OD) at 40. The resulting cell suspension was subjected to reaction and analysis in the same manner as described in Comparative Example 1. The results revealed that L-valine had an accumulated concentration of 4.3 mg/ml and an optical purity of 17% e.e.

Example 3:

The cell mass of Nocardia sp. PA-34 was prepared in the same manner as in Example 1, and was resuspended in a 0.1M $NH_4Cl$-$NH_3$ buffer solution so as to adjust the optical density (OD) at 40. Then, 500 ml of the resulting cell suspension was admitted in a 1.2-1 fermenter, to which 4.9 g (50 mmol) of DL-2-amino-3-methylbutanenitrile and 7.2 g (100 mmol) of isobutyraldehyde were added. They were reacted under stirring for 48 hours at a temperature of 30°C, at 700 rpm agitation, and at 0.2 vvm aeration, while maintaining the pH at 10 ± 0.1 with 4N aqueous ammonia and 1N HCl.

After completion of the reaction, the reaction mixture was analyzed in the same manner as described in Example 2. As a result, it was found that the accumulated concentration of L-valine was 1.3 mg/ml, while its optical purity was 96% e.e.

Example 4:

Using each of the microorganisms given in Table 4, a cell suspension was prepared in the same manner as described in Example 1. Then, 5 ml of the cell suspension was admitted in a test tube having an inner diameter of 24 mm, to which 49 mg of DL-2-amino-3-methylbutanenitrile and 72 mg of isobutyraldehyde were added. The test tube was stopcocked and the contents were reacted for 48 hours at a temperature of 30°C under 300 rpm oscillation. The reaction mixture thus-obtained was treated and analyzed in the same manner as described in Example 2. The results are given in Table 4.

Table 4

| Strain | L-valine | |
|---|---|---|
| | Amount (mg/ml) | Optical purity (% e.e.) |
| Nocardia sp. PC-29 | 0.8 | 100 |
| Nocardia sp. AB-18 | 0.5 | 96 |
| Nocardia sp. BA-1 | 0.4 | 100 |
| Mycobacterium sp. AB-43 | 0.6 | 97 |

Example 5:

Using each of the microorganisms given in Table 5, a cell suspension was prepared in the same manner as described in Example 1. Reaction was carried out according to the procedure described in Example 4 except for the addition of 50 µl of DL-2-aminopentanenitrile in place of DL-2-amino-3-methylbutanenitrile and 60 µl of n-butyraldehyde to 5 ml of the cell suspension. The L-norvaline thus-formed was analyzed with HPLC using Asahipak Inertsil ODS and CHIRALPAK WE as column packings. The results are shown in Table 5.

Table 5

| | L-norvaline | |
|---|---|---|
| Strain | Amount (mg/ml) | Optical purity (% e.e.) |
| Mycobacterium sp. AB-43 | 3.4 | 92 |
| Nocardia sp. PA-34 | 3.5 | 98 |

Example 6:

Using each of the microorganisms given in Table 6, a cell suspension was prepared in the same manner as described in Example 1. Reaction was carried out following the procedure described in Example 4 except for the addition of 50 µl of DL-4-methyl-2-aminopentane nitrile and 60 µl of 3-methylbutyraldehyde to 5 ml of the cell suspension. The L-leucine thus-formed was analyzed as in the same manner as described in Example 5. The results are shown in Table 6.

Table 6

| | L-leucine | |
|---|---|---|
| Strain | Amount (mg/ml) | Optical purity (% e.e.) |
| Mycobacterium sp. AB-43 | 1.4 | 97 |
| Nocardia sp. PA-34 | 1.6 | 95 |

Example 7:

Using each of the microorganisms given in Table 7, a cell suspension was prepared in the same manner as described in Example 1. Reaction was carried out following the procedure described in Example 4 except for the addition of 50 µl of DL-2-aminohexanenitrile and 60 µl of n-valeraldehyde. The L-norleucine thus-formed was analyzed as described in Example 5. The results are shown in Table 7.

Table 7

| | L-norleucine | |
|---|---|---|
| Strain | Amount (mg/ml) | Optical purity (% e.e.) |
| Mycobacterium sp. AB-43 | 1.1 | 100 |
| Nocardia sp. PA-34 | 1.3 | 100 |

Example 8:

Three loopfuls of Corynebacterium sp. PA-15 strain were inoculated to 100 ml of an NBG medium in a 500-ml Sakaguchi flask [the NBG medium is a liquid medium prepared by adding deionized water to 10 g of "Lab-Lemco" powder (code L29: product of Oxoid Co., Ltd.), 10 g of Bacteriological peptone (code L37), 10 g of glucose and 5 g of sodium chloride to make the total volume 1,000 ml, adjusting the pH of the resulting liquid at 7.5 with 1N aqueous sodium hydroxide solution, and then sterilizing the liquid with heat at 120°C for 15 minutes in an autoclave]. The cells were cultured under oscillation (150 times/minutes) at 30°C for 48 hours.

The cell mass formed by the incubation was collected and washed in the same manner as described in Example 1, and resuspended in a 0.1M $NH_4Cl$-$NH_3$ buffer solution so as to adjust the optical density (OD) at 40.

The cell suspension was reacted with DL-2-amino-3-methylbutanenitrile in the presence of isobutyraldehyde in the same manner as described in Example 4. The accumulated concentration of L-valine was 0.3 mg/ml and its optical purity was 95% e.e.

Example 9:

Reaction was carried out in the same manner as described in Example 4 except that 50 µl of DL-2-aminopentanenitrile and 60 µl of n-butyraldehyde were added to 5 ml of each of the cell suspensions prepared according to the procedure given in Example 8. The L-norvaline thus-formed was analyzed as described in Example 5. The results are shown in Table 8.

Table 8

| | L-norvaline | |
|---|---|---|
| Strain | Amount (mg/ml) | Optical purity (% e.e.) |
| Nocardia sp. BA-1 | 2.7 | 100 |
| Corynebacterium sp. PA-15 | 2.7 | 84 |
| Corynebacterium sp. PC-3 | 2.8 | 82 |

Example 10:

Reaction was carried out in the same manner as described in Example 4 except that 50 µl of DL-4-methyl-2-aminopentanenitrile and 60 µl of 3-methylbutyraldehyde were added to 5 ml of each of the cell suspensions prepared according to the procedure given in Example 8. The L-leucine thus-formed was analyzed as described in Example 5. The results are shown in Table 9.

Table 9

| | L-leucine | |
|---|---|---|
| Strain | Amount (mg/ml) | Optical purity (% e.e.) |
| Nocardia sp. BA-1 | 0.6 | 85 |
| Corynebacterium sp. PA-15 | 1.2 | 94 |
| Corynebacterium sp. PC-3 | 1.1 | 91 |

Example 11:

Reaction was carried out in the same manner as described in Example 3 except that 50 µl of DL-2-aminohexanenitrile and 60 µl of n-valeraldehyde were added to 5 ml of each of the cell suspensions prepared according to the

procedure given in Example 8. The L-norleucine thus-formed was analyzed as discribed in Example 5. The results are shown in Table 10.

Table 10

| | L-leucine | |
|---|---|---|
| Strain | Amount (mg/ml) | Optical purity (% e.e.) |
| Corynebacterium sp. PA-15 | 0.7 | 100 |
| Corynebacterium sp. PC-3 | 0.7 | 100 |

Example 12:

Cell suspensions of the strains given in Table 11 were prepared according to the procedure described in Example 1. In a test tube with an inner diameter of 24 mm were admitted 5 ml of each cell suspension, to which 100 µl of DL-3-methyl-2-(N-2-methylpropylideneamino)butanenitrile were added. The test tube was stopcocked, and incubated at 30°C under oscillation at 300 rpm for 48 hours.

After completion of the reaction, the resulting reaction mixture was extracted twice with 5 ml of hexane. The remaining water phase was analyzed with HPLC as discribed in Example 2. The results are as shown in Table 11.

Table 11

| | L-valine | |
|---|---|---|
| Strain | Amount (mg/ml) | Optical purity (% e.e.) |
| Nocardia sp. PC-29 | 2.3 | 96 |
| Nocardia sp. PA-34 | 0.6 | 98 |
| Nocardiasp. AB-16 | 1.3 | 92 |
| Nocardia sp. BA-1 | 0.9 | 95 |
| Mycobacterium sp. AB-43 | 3.0 | 97 |
| Corynebacterium sp. PA-15 | 2.5 | 100 |
| Corynebacterium sp. PC-3 | 1.6 | 100 |

Example 13:

The procedure described in Example 12 was followed except for the addition of 50 mg of DL-4-methyl-2-(N-3-methylbutylideneamino)pentanenitrile to 5 ml of the cell suspension of each strain given in Table 12; the cell suspension was prepared according to the procedure described in Example 1. The produced L-leucine was analyzed as described in Example 5. The results are shown in Table 12.

Table 12

| | L-leucine | |
|---|---|---|
| Strain | Amount (mg/ml) | Optical purity (% e.e.) |
| Nocardia sp. PA-34 | 2.5 | 93 |
| Mycobacterium sp. AB-43 | 0.6 | 83 |

Example 14:

The procedure described in Example 13 was followed except for the addition of 50 µl of DL-2-(N-butylideneamino) pentanenitrile to 5 ml of the cell suspension of each strain given in Table 13, the cell suspension being prepared according to the procedure described in Example 1. The results are shown in Table 13.

Table 13

| | L-norvaline | |
|---|---|---|
| Strain | Amount (mg/ml) | Optical purity (% e.e.) |
| Nocardia sp. AB-16 | 2.6 | 90 |
| Mycobacterium sp. AB-43 | 2.4 | 66 |
| Nocardia sp. PA-34 | 2.1 | 90 |
| Nocardia sp. PC-29 | 1.5 | 60 |

Example 15:

The procedure described in Example 13 was followed except for the addition of 50 μl of DL-2-(N-pentylideneamino) hexanenitrile to 5 ml of the cell suspension of each strain given in Table 14, the cell suspension being prepared according to the procedure described in Example 1. The results are shown in Table 14.

Table 14

| | L-norleucine | |
|---|---|---|
| Strain | Amount (mg/ml) | Optical purity (% e.e.) |
| Nocardia sp. AB-16 | 1.3 | 98 |
| Mycobacterium sp. AB-43 | 0.8 | 86 |
| Nocardia sp. PA-34 | 1.1 | 96 |
| Nocardia sp. PC-29 | 0.5 | 51 |

Example 16:

A cell suspension of Nocardia sp. PA-34 was prepared according to the procedure described in Example 1 except that each of the nitrile compounds given in Table 15 was used in place of propionitrile. To 5 ml of the cell suspension were added 50 μl of DL-3-methyl-2-(N-2-methylpropylideneamino)butanenitrile. Reaction and analysis were conducted following the procedure described in Example 12. The results are given in Table 15.

Table 15

| | L-valine | |
|---|---|---|
| Nitrile compound | Amount (mg/ml) | Optical purity (% e.e.) |
| isobutyronitrile | 0.8 | 94 |
| glutaronitrile | 0.9 | 100 |
| adiponitrile | 0.1 | 100 |
| n-butyronitile | 0.6 | 85 |
| methacrylonitrile | 0.3 | 100 |
| crotononitrile | 0.6 | 74 |

Example 17:

Three loopfuls of each of the microorganisms given in Table 16 were inoculated to 100 ml of an NBG medium in a 500-ml flask. The flask was oscillated (150 times/minute) at 30°C for 48 hours to incubate the cells. The cell mass grown by the incubation was collected and washed in the same manner as described in Example 1 and resuspended in a 0.1M $NH_4Cl$-$NH_3$ buffer solution so as to adjust the optical density (OD) at 40. The cell suspension was reacted with 50 μl of DL-2-(N-butylideneamino)pentanenitrile in the same manner as described in Example 13. The results are shown in Table 16.

Table 16

| | L-norvaline | |
|---|---|---|
| Strain | Amount (mg/ml) | Optical purity (% e.e.) |
| Nocardia sp. BA-1 | 0.1 | 80 |
| Corynebacterium sp. PA-15 | 1.8 | 94 |
| Corynebacterium sp. PC-3 | 1.5 | 91 |

Example 18:

Reaction was conducted in the same manner as described in Example 17 except for the addition of 50 µl of DL-2-(N-pentylideneamino)hexanenitrile to 5 ml of the cell suspension of each strain given in Table 17; the cell suspension was prepared according to the procedure described in Example 17. The results are shown in Table 17.

Table 17

| | L-norleucine | |
|---|---|---|
| Strain | Amount (mg/ml) | Optical purity (% e.e.) |
| Corynebacterium sp. PA-15 | 0.7 | 84 |
| Corynebacterium sp. PC-3 | 0.5 | 60 |

Example 19:

Reaction was conducted in the same manner as described in Example 17 except for the addition of 50 µl of DL-4-methyl-2-(N-3-methylbutylideneamino)pentenenitrile to 5 ml of the cell suspension of each strain given in Table 18; the cell suspension was prepared according to the procedure described in Example 17. The results are shown in Table 18.

Table 18

| | L-leucine | |
|---|---|---|
| Strain | Amount (mg/ml) | Optical purity (% e.e.) |
| Corynebacterium sp. PA-15 | 0.6 | 100 |
| Corynebacterium sp. PC-3 | 0.5 | 100 |

**Claims**

1. A process for the production of an L-α-amino acid which comprises causing a microorganism having enantioselective nitrile-hydrolysing activity to act at a pH in the range of 8-12 on one or more α-aminonitrile compounds represented by the following general formula (I) and/or (II):

$$R - \overset{NH_2}{CH} - CN \qquad (I)$$

$$R - CH_2\overset{NH_2}{CH} - CN \qquad (II)$$

wherein R is an alkyl group, substituted alkyl group, phenyl group, substituted phenyl group, imidazolyl group, substituted imidazolyl group, indolyl group, substituted indolyl group, furyl group, substituted furyl group, pyridyl group, substituted pyridyl group, thiazolyl group or substituted thiazolyl group.

2. A process for the production of an L-α-amino acid which comprises causing a microorganism having enantioselective nitrile-hydrolysing activity to act in the presence of an aldehyde on one or more α-aminonitrile compounds

represented by the following general formal (I) and/or (II):

$$\begin{matrix} & NH_2 & \\ R - & CH & - CN \end{matrix} \qquad (I)$$

$$\begin{matrix} & NH_2 & \\ R - & CH_2CH & - CN \end{matrix} \qquad (II)$$

wherein R is an alkyl group, substituted alkyl group, phenyl group, substituted phenyl group, imidazolyl group, substituted imidazolyl group, indolyl group, substituted indolyl group, furyl group, substituted furyl group, pyridyl group, substituted pyridyl group, thiazolyl group or substituted thiazolyl group,

wherein there is 0.1 to 10 mols of aldehyde per mol of the α-aminonitrile compound(s).

**3.** A process for the production of an L-α-amino acid which comprises causing a microorganism having nitrile-hydrolysing activity to act on one or more nitrile compounds represented by the following general formula (III):

$$\begin{matrix} N = CH - R' \\ R - CH - CN \end{matrix} \qquad (I)$$

and/or the following general formula (IV):

$$\begin{matrix} N = CH - CH_2R' \\ R - CH_2CH = CN \end{matrix} \qquad (IV)$$

wherein R and R' are individually an alkyl group,
substituted alkyl group, phenyl group, substituted phenyl group, imidazolyl group, substituted imidazolyl group, indolyl group, substituted indolyl group, furyl group, substituted furyl group, pyridyl group, substituted pyridyl group, thiazolyl group or substituted thiazolyl group,

**4.** The process for the production of an L-α-amino acid as claimed in any one of claims 1, 2 or 3 wherein the microorganism is FERM-BP No 1561, FERM-BP No 1559, FERM-BP No 1555 or FERM-BP No 1557.

**5.** The process for the production of an L-α-amino acid as claimed in any one of claims 1,2 or 3 wherein the microorganism is Mycobacterium sp. AB-43 (FERM BP No 1556).

**6.** The process for the production of an L-α-amino acid as claimed in any one of claims 1, 2 or 3 wherein the microorganism is FERM-BP No 1558 or FERM-BP No 1560.

**7.** The process for the production of an L-α-amino acid as claimed in claim 1, 2 or 3 wherein the reaction is conducted in an ammoniacal buffer solution.

**8.** The process for the production of an L-α-amino acid as claimed in claim 7 wherein the ammoniacal buffer solution has a pH in the range of 8-12.

**Patentansprüche**

**1.** Verfahren zur Herstellung einer L-α-Aminosäure, welches das Einwirken eines Mikroorganismus mit enantioselektiver Nitril-Hydrolyseaktivität bei einem pH-Wert im Bereich von 8-12 auf eine oder mehrere α-Aminonitrilverbindungen umfaßt, die durch die folgende allgemeine Formel (I) und/oder (II) dargestellt sind:

$$\begin{array}{c} NH_2 \\ R - CH - CN \end{array} \qquad (I)$$

$$\begin{array}{c} NH_2 \\ R - CH_2CH - CN \end{array} \qquad (II)$$

worin R eine Alkylgruppe, eine substituierte Alkylgruppe, eine Phenylgruppe, eine substituierte Phenylgruppe, eine Imidazolylgruppe, eine substituierte Imidazolylgruppe, eine Indolylgruppe, eine substituierte Indolylgruppe, eine Furylgruppe, eine substituierte Furylgruppe, eine Pyridylgruppe, eine substituierte Pyridylgruppe, eine Thiazolylgruppe oder eine substituierte Thiazolylgruppe ist.

**2.** Verfahren zur Herstellung einer L-α-Aminosäure, welches das Einwirken eines Mikroorganismus mit enantioselektiver Nitril-Hydrolyseaktivität in Gegenwart eines Aldehyds auf eine oder mehrere α-Aminonitrilverbindungen umfaßt, die durch die folgende allgemeine Formel (I) und/oder (II) dargestellt sind:

$$\begin{array}{c} NH_2 \\ R - CH - CN \end{array} \qquad (I)$$

$$\begin{array}{c} NH_2 \\ R - CH_2CH - CN \end{array} \qquad (II)$$

worin R eine Alkylgruppe, eine substituierte Alkylgruppe, eine Phenylgruppe, eine substituierte Phenylgruppe, eine Imidazolylgruppe, eine substituierte Imidazolylgruppe, eine Indolylgruppe, eine substituierte Indolylgruppe, eine Furylgruppe, eine substituierte Furylgruppe, eine Pyridylgruppe, eine substituierte Pyridylgruppe, eine Thiazolylgruppe oder eine substituierte Thiazolylgruppe ist,

und wobei 0,1 bis 10 Mol Aldehyd pro Mol α-Aminonitrilverbindung(en) vorliegen.

**3.** Verfahren zur Herstellung einer L-α-Aminosäure, welches das Einwirken eines Mikroorganismus mit Nitril-Hydrolyseaktivität auf eine oder mehrere Nitrilverbindungen umfaßt, die durch die folgende allgemeine Formel (III):

$$\begin{array}{c} N = CH - R' \\ R - CH - CN \end{array} \qquad (III)$$

und/oder die folgende allgemeine Formel (IV) dargestellt sind:

$$\begin{array}{c} N = CH - CH_2R' \\ R - CH_2CH = CN \end{array} \qquad (IV)$$

worin R und R' jeweils eine Alkylgruppe, eine substituierte Alkylgruppe, eine Phenylgruppe, eine substituierte Phenylgruppe, eine Imidazolylgruppe, eine substituierte Imidazolylgruppe, eine Indolylgruppe, eine substituierte Indolylgruppe, eine Furylgruppe, eine substituierte Furylgruppe, eine Pyridylgruppe, eine substituierte Pyridylgruppe, eine Thiazolylgruppe oder eine substituierte Thiazolylgruppe sind.

**4.** Verfahren zur Herstellung einer L-α-Aminosäure nach einem der Ansprüche 1, 2 oder 3, wobei der Mikroorganismus FERM-BP Nr. 1561, FERM-BP Nr. 1559, FERM-BP Nr. 1555 oder FERM-BP Nr. 1557 ist.

**5.** Verfahren zur Herstellung einer L-α-Aminosäure nach einem der Ansprüche 1, 2 oder 3, wobei der Mikroorganismus Mycobacterium sp. AB-43 (FERM-BP Nr. 1556) ist.

**6.** Verfahren zur Herstellung einer L-α-Aminosäure nach einem der Ansprüche 1, 2 oder 3, wobei der Mikroorganismus FERM-BP Nr. 1558 oder FERM-BP Nr. 1560 ist.

**7.** Verfahren zur Herstellung einer L-α-Aminosäure nach einem der Anspüche 1, 2 oder 3, wobei die Reaktion in einer ammoniakalischen Pufferlösung durchgeführt wird.

**8.** Verfahren zur Herstellung einer L-α-Aminosäure nach Anspruch 7, wobei die ammoniakalische Pufferlösung einen pH-Wert im Bereich von 8-12 aufweist.

## Revendications

**1.** Procédé pour la production d'un L-α-aminoacide qui consiste à faire agir un micro-organisme, ayant une activité d'hydrolyse de nitrile énantio-sélective, à un pH compris entre 8 et 12 sur un ou plusieurs composés α-aminonitrile représentés par la formule générale (I) et/ou (II):

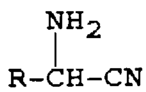 (I)

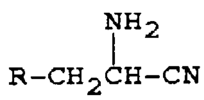 (II)

dans lesquelles R représente un groupe alkyle, un groupe alkyle substitué, un groupe phényle, un groupe phényle substitué, un groupe imidazolyle, un groupe imidazolyle substitué, un groupe indolyle, un groupe indolyle substitué, un groupe furyle, un groupe furyle substitué, un groupe pyridyle, un groupe pyridyle substitué, ou groupe thiazolyle ou un groupe thiazolyle substitué.

**2.** Procédé pour la production d'un L-α-aminoacide qui consiste à faire agir un micro-organisme, ayant une activité d'hydrolyse de nitrile énantio-sélective, en présence d'un aldéhyde sur un ou plusieurs composés α-aminonitrile représentés par la formule générale (I) et/ou (II) suivantes:

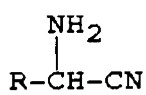 (I)

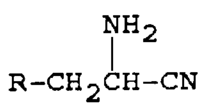 (II)

dans lesquelles R représente un groupe alkyle, un groupe alkyle substitué, un groupe phényle, un groupe phényle substitué, un groupe imidazolyle, un groupe imidazolyle substitué, un groupe indolyle, un groupe indolyle substitué, un groupe furyle, un groupe furyle substitué, un groupe pyridyle, un groupe pyridyle substitué, un groupe thiazolyle ou un groupe thiazolyle substitué

dans lequel il y a 0,1 à 10 moles d'aldéhyde par mole du ou des composés α-aminonitrile.

**3.** Procédé pour la production d'un L-α-aminoacide qui consiste à faire agir un micro-organisme, ayant une activité d'hydrolyse de nitrile, sur un ou plusieurs composés nitrile représentés par la formule générale (III) suivante :

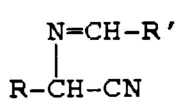 (III)

et/ou la formule générale (IV) suivante:

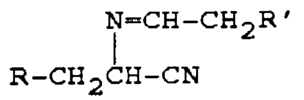 (IV)

dans lesquelles R et R' représentent individuellement un groupe alkyle, un groupe alkyle substitué, un groupe phényle, un groupe phényle substitué, un groupe imidazolyle, un groupe imidazolyle substitué, un groupe indolyle, un groupe indolyle substitué, un groupe furyle, un groupe furyle substitué, un groupe pyridile, un groupe pyridyle substitué, un groupe thiazolyle ou un groupe thiazolyle substitué.

**4.** Procédé pour la production d'un L-α-amino-acide selon l'une quelconque des revendications 1, 2 ou 3 dans lequel le micro-organisme est FERM-BP n°1561, FERM n° 1559, FERM-BP n° 1555 ou FERM-BP n° 1557.

**5.** Procédé pour la production d'un L-α-amino-acide selon l'une quelconque des revendications 1, 2 ou 3 dans lequel le micro-organisme est Mycobacterium espèce AB-43 (FERM BP n° 1556).

**6.** Procédé pour la production d'un L-α-amino-acide selon l'une quelconque des revendications 1, 2 ou 3, dans lequel le micro-organisme est FERM-BP n° 1558 ou FERM-BP n°1560.

**7.** Procédé pour la production d'un L-α-amino-acide selon la revendication 1, 2 ou 3, dans lequel la réaction est conduite dans une solution de tampon ammoniacal.

**8.** Procédé pour la production d'un L-α-amino-acide selon la revendication 7, dans lequel la solution de tampon ammoniacal a un pH compris entre 8 et 12.